# EUROPÄISCHE PATENTSCHRIFT

(11) **EP 1 014 875 B1**
(45) Veröffentlichungstag und Bekanntmachung des Hinweises auf die Patenterteilung: **07.04.2004**
(21) Anmeldenummer: 98948964.6
(22) Anmeldetag: 11.09.1998
(51) Int. Cl.: A61B 17/32

(54) **CHIRURGISCHES ROHRSCHAFTINSTRUMENT**
SURGICAL INSTRUMENT WITH A TUBULAR STEM
INSTRUMENT CHIRURGICAL A TIGE TUBULAIRE

(30) Priorität: 18.09.1997 DE 19741054
(43) Veröffentlichungstag der Anmeldung: 05.07.2000
(73) Patentinhaber: Aesculap AG & Co. KG, 78532 Tuttlingen (DE)
(72) Erfinder: MORALES, Pedro, D-78532 Tuttlingen (DE); NESPER, Markus, D-78532 Tuttlingen (DE); WEISSHAUPT, Dieter, D-78194 Immendingen (DE)
(74) Vertreter: Böhme, Ulrich, Dr. Dipl.-Phys.
(86) Internationale Anmeldenummer: PCT/EP1998/005805
(87) Internationale Veröffentlichungsnummer: WO 1999/013787

(56) Entgegenhaltungen:
- EP-A- 0 479 482
- DE-A- 3 217 105
- DE-A- 4 113 037
- DE-U- 9 318 815

## Beschreibung

Die Erfindung betrifft ein chirurgisches Rohrschaftinstrument mit einem Schaft, an dessen distalem Ende mindestens ein Werkzeug an einem Halter angeordnet ist, der seinerseits am Schaft festgelegt ist, und mit einer den Halter teilweise abdeckenden Kunststoffummantelung.

Ein derartiges Rohrschaftinstrument ist beispielsweise aus der DE 93 18 815 U1 bekannt. Mit einem solchen Rohrschaftinstrument ist es möglich, einerseits sehr solide metallische Werkstoffe für die Ausgestaltung des Halters und des Schaftes zu verwenden und andererseits den Schaft einschließlich des Halters bis in den Bereich der Werkzeuge nach außen hin elektrisch zu isolieren, einmal dadurch, daß der Schaft selber von einer elektrisch isolierenden Hülse umgeben ist, zum anderen dadurch, daß der Halter bereichsweise eine Kunststoffummantelung aufweist, die nach außen weisende Bereiche des metallischen Halters abdeckt. Setzt man ein solches Rohrschaftinstrument als elektrisches Schneid- und Koagulationsinstrument ein, so ist auf diese Weise gewährleistet, daß eine an das Instrument angelegte Spannung nur im Bereich des eigentlichen Werkzeuges mit dem Körpergewebe in Kontakt kommt, so daß nur in diesem Bereich ein Schneiden oder Koagulieren auftritt.

In der Praxis hat sich herausgestellt, daß derartige Kunststoffummantelungen in ihrem Randbereich im Laufe der Zeit vom Halter abheben und in diesem Bereich Hohlräume ausbilden, die verschmutzen und die sehr schwer gereinigt werden können. Dies liegt einmal daran, daß sich das Material der Kunststoffummantelung, die üblicherweise durch Umspritzen aufgebracht wird, nicht mit dem Material des Halters verbindet, beispielsweise kann dieses Material Edelstahl sein. Andererseits dringt beim Sterilisieren dieser Rohrschaftinstrumente Dampf gegebenenfalls unter erhöhtem Druck in die Spalte zwischen der Kunststoffummantelung und dem Halter ein und vergrößert diese Spalte, so daß das Kunststoffmaterial der Kunststoffummantelung von seiner flächigen Anlage am Halter entfernt wird.

Es ist Aufgabe der Erfindung, ein gattungsgemäßes Rohrschaftinstrument so auszugestalten, daß ein solches Abheben der Kunststoffummantelung vom Halter und insbesondere die Ausbildung von größeren, schwer zu reinigenden Hohlräumen verhindert werden.

Diese Aufgabe wird bei einem chirurgischen Rohrschaftinstrument der eingangs beschriebenen Art erfindungsgemäß dadurch gelöst, daß die Kunststoffummantelung an ihrem Rand Vorsprünge am Halter untergreift. Der Anschluß zwischen der Kunststoffummantelung einerseits und dem Halter andererseits wird also so ausgebildet, daß der Halter die benachbarte Kunststoffummantelung im Randbereich übergreift und dadurch ein Abheben von der Unterlage des Halters verhindert, man erhält auf diese Weise praktisch eine formschlüssige Festlegung der Kunststoffummantelung im Randbereich.

Bei einer bevorzugten Ausführungsform ist vorgesehen, daß die Vorsprünge durch Hinterschneidungen einer Stufe im Halter gebildet werden, die einen tieferliegenden, von der Kunststoffummantelung bedeckten Bereich des Halters begrenzt. Eine solche Stufe kann sich beispielsweise über den gesamten Umfang des Halters erstrecken und führt dazu, daß die Kunststoffummantelung längs ihres gesamten Randes in gleicher Weise am Halter festgelegt wird.

Dabei ist es vorteilhaft, wenn der rückspringende Hohlraum der Hinterschneidung vom Material der Kunststoffummantelung vollständig ausgefüllt ist. Dies ergibt einen vollständigen Formschluß und verhindert, daß sich an irgendeiner Stelle Hohlräume ausbilden, die verschmutzt werden können.

Besonders vorteilhaft ist es, wenn die Hinterschneidung durch eine schräg zur Stufenhöhe verlaufende Grenzfläche der Stufe gebildet wird.

Gemäß einer bevorzugten Ausführungsform ist vorgesehen, daß die Kunststoffummantelung an nicht ummantelte benachbarte Bereiche des Halters stufenlos anschließt, so daß man eine durchgehende Außenfläche erhält, die sich über den Spalt zwischen Halterfläche und Kunststoffummantelungsfläche stetig erstreckt.

Das Material der Kunststoffummantelung wird vorzugsweise ein dampfsterilisierbarer Kunststoff sein, beispielsweise Polyetheretherketon (PEEK) oder Liquid Cristal Polymere (LCP).

Die nachfolgende Beschreibung bevorzugter Ausführungsformen der Erfindung dient im Zusammenhang mit der Zeichnung der näheren Erläuterung. Es zeigen:

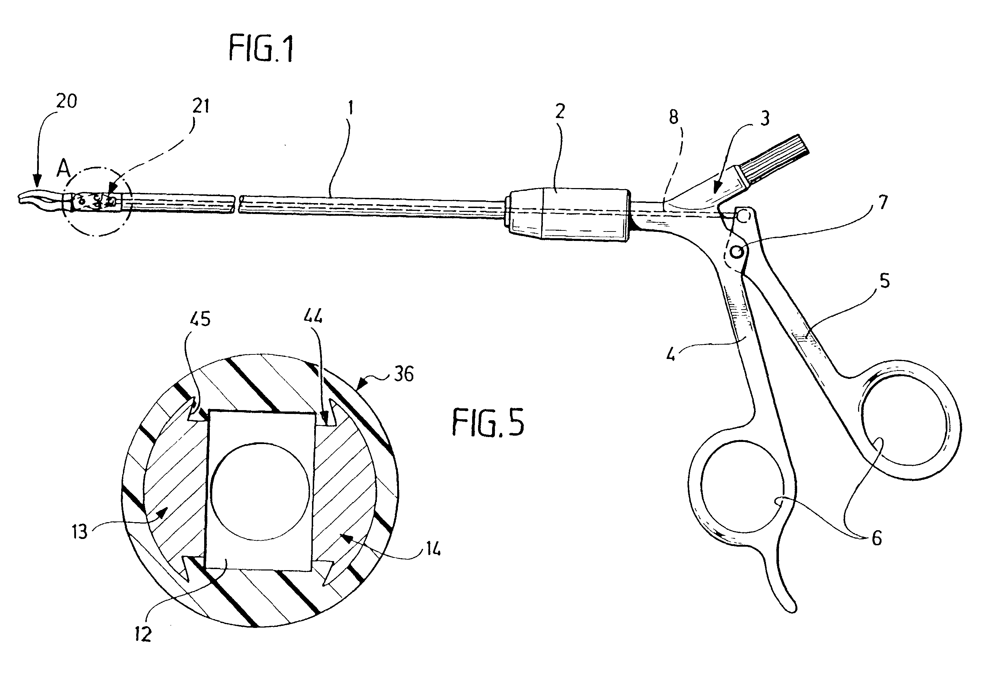

- Figur 1: eine Seitenansicht eines Rohrschaftinstrumentes;

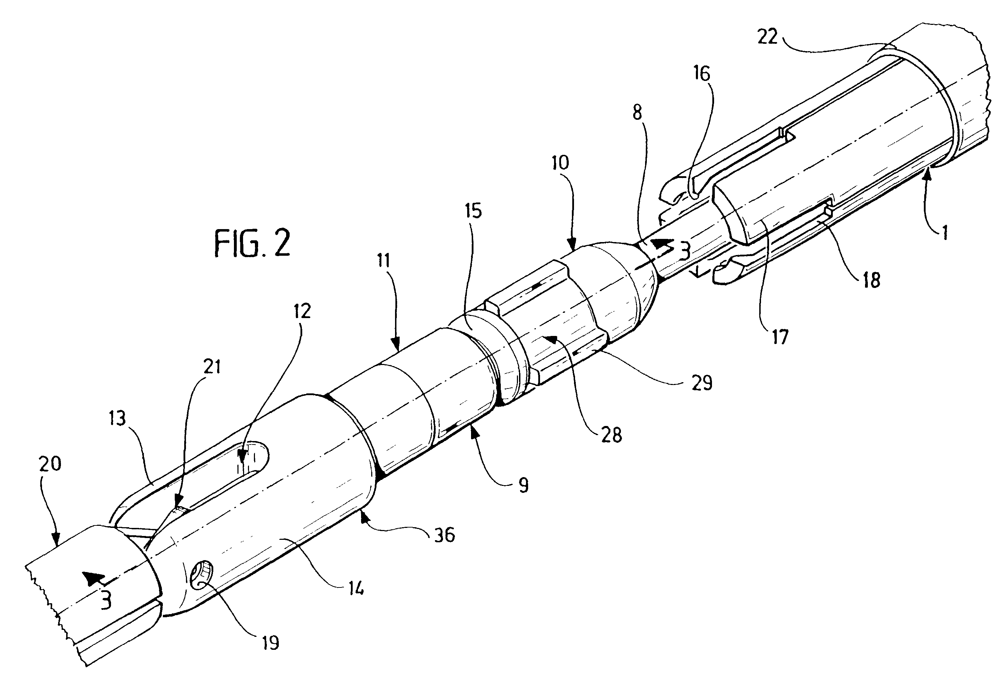

- Figur 2: eine perspektivische Teilansicht des Bereiches A in Figur 1 bei in Längsrichtung auseinandergezogenem Rohrschaftinstrument;
- Figur 3: eine Längsschnittansicht des Halters der Figur 2;
- Figur 4: eine Seitenansicht des Halters der Figur 3 in um 90° gedrehter Stellung mit teilweise aufgebrochenen Bereichen und
- Figur 5: eine Schnittansicht längs Linie 5-5 in Figur 4.

Das in der Zeichnung dargestellte Rohrschaftinstrument umfaßt einen rohrförmigen Schaft 1, der an seinem proximalen Ende über ein Kupplungsstück 2 mit einem Griffteil 3 lösbar verbunden ist. Das Griffteil 3 weist zwei Branchen 4, 5 mit Fingeröffnungen 6 auf, die um eine Schwenkachse 7 gegeneinander verschwenkt werden können. Die eine Branche 4 ist mit dem Schaft 1 verbunden, die andere Branche 5 gelenkig mit einem stabförmigen Betätigungselement 8, welches im Inneren des Schaftes 1 angeordnet ist und sich bis zu dessen distalem Ende erstreckt. Beim Verschwenken der Branchen 4 und 5 gegeneinander wird das Betätigungselement 8 gegenüber dem Schaft 1 in Längsrichtung verschoben.

Am distalen Ende greift in den Schaft 1 ein rohrförmiger Halter 9 ein, der an seinem dem Schaft 1 zugewandten Ende einen Einschubnippel 10, daran anschließend einen zylindrischen Abschnitt 11 und daran anschließend zwei parallel zueinander verlaufende, zwischen sich einen Aufnahmeraum 12 ausbildende Längsarme 13 und 14 aufweist.

Der Einschubnippel 10 ist in das distale Ende des Schaftes 1 einschiebbar, an seinem Übergangsbereich zum zylindrischen Abschnitt 11 weist er eine Umfangsnut 15 auf, in die die nach innen vorspringenden Enden 16 der Wandabschnitte 17 des Schaftes 1 eintauchen können. Diese Wandabschnitte 17 sind durch Längsschnitte 18 in voneinander unabhängige Bereiche unterteilt, so daß diese Wandabschnitte 17 radial nach innen oder außen biegbare Federzungen ausbilden, die einstückig mit dem aus einem entsprechenden elastischen Material bestehenden Schaft 1 verbunden sind.

Die Längsarme 13 und 14 weisen in der Nähe ihres freien Endes quer zur Längsrichtung des Halters 9 verlaufende Öffnungen 19 auf, in die eine in der Zeichnung nicht dargestellte Lagerwelle von Werkzeugen 20 einsetzbar ist, so daß die Werkzeuge 20 um die durch die Lagerwelle gebildete Achse verschwenkbar im Aufnahmeraum 12 gehalten sind und aus diesem in distaler Richtung hervorstehen. Diese Werkzeuge können beispielsweise Scherenblätter, Klemmbacken oder ähnliches sein. Diese Werkzeuge 20 werden über Getriebemittel 21 über das Betätigungselement 8 verschwenkt, wenn dieses in Längsrichtung gegenüber dem Schaft 1 verschoben wird. Hier können an sich bekannte Techniken verwendet werden, so daß diese Getriebemittel 21 hier nicht näher erläutert werden.

Auf dem Schaft 1 ist in Längsrichtung verschiebbar eine Außenhülse 22 gelagert, die aus elektrisch isolierendem Material besteht. Im vorgeschobenen Zustand überdeckt sie die federnden Wandabschnitte 17 des Schaftes 1 und hält somit deren nach innen vorspringende Enden 16 in der Umfangsnut 15 des Einschubnippels 10, dadurch wird der Halter 9 im Schaft 1 in axialer Richtung unverschieblich gehalten. Zur Lösung dieser Verbindung genügt es, die Außenhülse 22 zurückzuschieben, so daß die Wandabschnitte 17 nach außen federn können, dabei treten ihre Enden 16 aus der Umfangsnut 15 aus, und der Halter kann dann aus dem Schaft 1 herausgezogen werden.

Durch die Verwendung eines elektrisch isolierenden Materials deckt die Außenhülse 22 außerdem bei eingesetztem Halter den gesamten Schaft 1 und den zylindrischen Abschnitt 11 des Halters 9 ab.

Im Einschubnippel 10 ist eine breite Umfangsnut 23 ausgebildet, die seitlich durch je eine Stufe 24 begrenzt wird. Diese Stufe ist im Querschnitt durch eine schräg verlaufende Grenzfläche 25 hinterschnitten ausgebildet. In den Boden 26 der Umfangsnut 23 ist außerdem eine schmale Umfangsnut 27 mit im wesentlichen dreieckförmiger Querschnittsfläche eingearbeitet.

Die Umfangsnut 23 nimmt eine diese vollständig ausfüllende Kunststoffummantelung 28 auf, die über die Außenfläche des Einschubnippels 10 radial vorstehende Längsrippen 29 trägt. Diese Längsrippen gleiten beim Einschieben des Einschubnippels 10 in den Schaft 1 in die Längsschnitte 18 zwischen den Wandabschnitten 17 und bilden eine Verdrehsicherung für den Halter 9. In dem zwischen den Längsrippen 29 liegenden Bereich schließt sich die Kunststoffummantelung 28 stufenlos an die Umfangsfläche des Einschubnippels 10 in den neben der Umfangsnut 23 liegenden Bereichen an.

Die Kunststoffummantelung 28 kann beispielsweise durch Umspritzen des Halters im Bereich der Umfangsnut 23 aufgebracht werden. Das in dieser Weise aufgebrachte Kunststoffmaterial hintergreift die eine Hinterschneidung bildende Stufe 24, so daß im Bereich des Randes der Kunststoffummantelung 28 eine formschlüssige Verbindung zwischen Kunststoffummantelung 28 einerseits und Einschubnippel 10 andererseits gebildet wird, durch die verhindert wird, daß sich die Kunststoffummantelung 28 in radialer Richtung vom Boden 26 der Umfangsnut 23 abheben kann. Durch das Eingreifen des Materials der Kunststoffummantelung 28 in die Umfangsnut 27 wird zusätzlich eine axiale Sicherung erzielt.

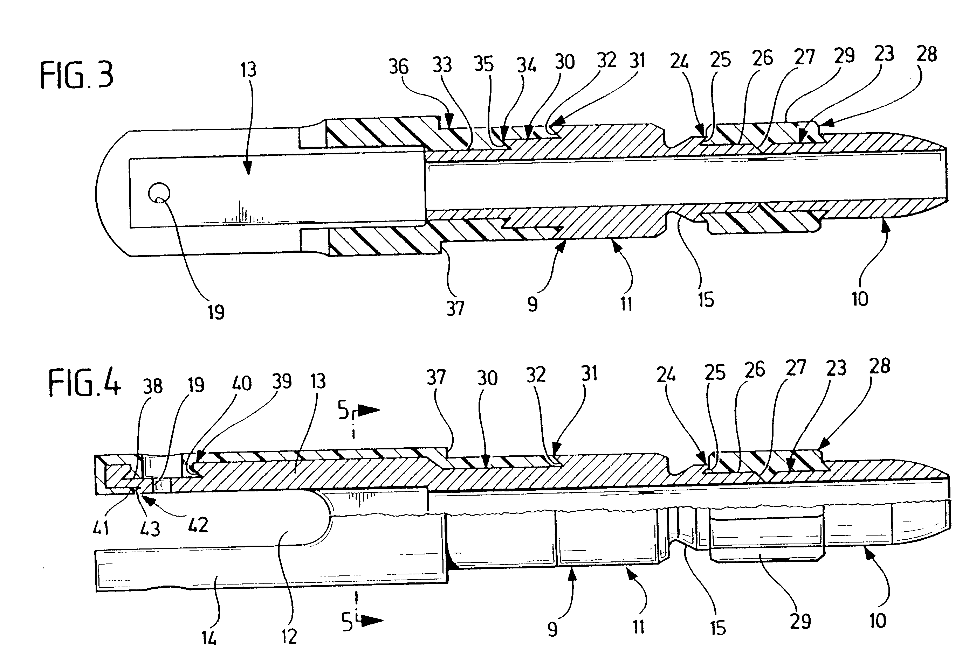

In dem zylindrischen Abschnitt 11 befindet sich ebenfalls eine Umfangsnut 30, die sich etwa von der Mitte des zylindrischen Abschnittes 11 bis zum Ansatz der Längsarme 13, 14 erstreckt. Diese Umfangsnut 30 wird an ihrem distalen Ende durch eine Stufe 31 begrenzt, die ebenfalls hinterschnitten ist und eine schräge Grenzfläche 32 aufweist. Über einen Teil des Umfanges ist in diese Umfangsnut 30 eine weitere in Umfangsrichtung verlaufende Nut 33 eingelassen, die eine Vertiefung im Boden der Umfangsnut 30 bildet und die ebenfalls durch eine hinterschnittene Stufe 34 mit einer schrägen Grenzfläche 35 begrenzt wird (Figur 3).

Die Umfangsnut 30 und die Nut 33 sind vollständig ausgefüllt von einer weiteren Kunststoffummantelung 36, die im Bereich der Stufe 31 stufenlos in die Umfangsfläche des zylindrischen Abschnittes 11 übergeht. Im Übergangsbereich zwischen dem zylindrischen Abschnitt 11 und den Längsarmen 13, 14 bildet diese Kunststoffummantelung 36 eine radial nach außen springende Stufe 37 aus, die einen Anschlag für die Außenhülse 22 bildet.

Die Kunststoffummantelung 36 setzt sich an der Außenseite der Längsarme 13 und 14 fort und umgibt diese an der Oberseite, an der Außenseite und an der Unterseite sowie an der Stirnseite und am distalen Ende der Innenseite.

Im Bereich der Öffnungen 19 weisen die Längsarme 13 und 14 zylindrische Vertiefungen 38 auf, deren Außenrand eine hinterschnittene Stufe 39 mit einer schrägen Grenzfläche 40 ausbildet. Die Kunststoffummantelung 36 greift in diese Vertiefung 38 ein, läßt jedoch den zentralen Bereich der Vertiefung 38 frei, so daß die Öffnung 19 von außen her zugänglich bleibt.

Der an der Innenseite der Längsarme liegende Rand 41 der Kunststoffummantelung 36 untergreift eine ebenfalls hinterschnittene Stufe 42 mit einer schrägen Grenzfläche 43, die zwischen dem distalen Ende der Längsarme 13, 14 einerseits und der Öffnung 19 andererseits quer zur Längsrichtung der Längsarme 13, 14 verläuft.

Auf diese Weise ist sichergestellt, daß die Kunststoffummantelung im Bereich der Stufen 31, 34 sowie 39 und 42 formschlüssig am Halter 9 gehalten und gegen ein Ablösen gesichert ist.

Eine ähnliche Sicherung ergibt sich an der Oberseite und an der Unterseite der Längsarme 13 und 14, diese weisen nämlich zu ihrer Innenseite hin parallel zur Längsrichtung der Längsarme 13, 14 verlaufende, zur Innenseite hin offene Längsnuten 44 auf, deren Seitenwände 45 hinterschnitten sind, und zwar durch eine schräge Ausbildung der Grenzflächen 45. Auch diese Längsnuten 44 mit den hinterschnittenen Grenzflächen 45 werden von dem Material der Kunststoffummantelung 36 ausgefüllt und dadurch formschlüssig gegen ein Ablösen gesichert.

Die Kunststoffummantelung 36 ist in dem dem zylindrischen Abschnitt 11 benachbarten Bereich in sich geschlossen ausgebildet, wie dies in Figur 5 dargestellt ist. Dies gilt für den Bereich der Längsarme 13 und 14, in die sich der Aufnahmeraum 12 nicht hineinerstreckt. Im Bereich des Aufnahmeraumes 12 hingegen bleibt dieser in der Kunststoffummantelung 36 seitlich ausgespart, so daß in den Aufnahmeraum 12 eintauchende Werkzeuge seitlich aus dem Aufnahmeraum 12 herausgeschwenkt werden können.

## Patentansprüche

1. Chirurgisches Rohrschaftinstrument mit einem Schaft (1), an dessen distalem Ende mindestens ein Werkzeug (20) an einem Halter (9) angeordnet ist, der seinerseits am Schaft (1) festgelegt ist, und mit einer den Halter (9) teilweise abdeckenden Kunststoffummantelung (28; 36), **dadurch gekennzeichnet, daß** die Kunststoffummantelung (28; 36) an ihrem Rand Vorsprünge (24; 31, 34, 39, 41) am Halter (9) untergreift.

2. Instrument nach Anspruch 1, **dadurch gekennzeichnet, daß** die Vorsprünge durch Hinterschneidungen einer Stufe (24; 31, 34, 39, 41) im Halter (9) gebildet werden, die einen tieferliegenden, von der Kunststoffummantelung (28; 36) bedeckten Bereich (23; 30, 38) des Halters (9) begrenzt.

3. Instrument nach Anspruch 2, **dadurch gekennzeichnet, daß** der rückspringende Hohlraum der Hinterschneidung vom Material der Kunststoffummantelung (28; 36) vollständig ausgefüllt ist.

4. Instrument nach einem der Ansprüche 2 oder 3, **dadurch gekennzeichnet, daß** die Hinterschneidung durch eine schräg zur Stufenhöhe verlaufende Grenzfläche (25; 32, 35, 40, 43, 45) der Stufe gebildet wird.

5. Instrument nach einem der voranstehenden Ansprüche, **dadurch gekennzeichnet, daß** die Kunststoffummantelung (28; 36) an nicht ummantelte benachbarte Bereiche des Halters (9) stufenlos anschließt.

6. Instrument nach einem der voranstehenden Ansprüche, **dadurch gekennzeichnet, daß** das Material der Kunststoffummantelung (28; 36) Polyetheretherketon (PEEK) ist.

7. Instrument nach einem der Ansprüche 1 bis 5, **dadurch gekennzeichnet, daß** das Material der Kunststoffummantelung (28; 36) Liquid Cristal Polymere (LCP) ist.

## Claims

1. A tubular-shaft surgical instrument comprising a shaft (1), at the distal end of which at least one implement (20) is arranged on a holder (9) which, for its part, is fixed to the shaft (1), and comprising a plastics sheathing (28; 36) partly covering the holder (9), **characterised in that** the plastics sheathing (28; 36) engages, at its edge, below projections (24; 31, 34, 39, 41) on the holder (9).

2. An instrument according to claim 1, **characterised in that** the projections are formed by undercuts of a step (24; 31, 34, 39, 41) provided in the holder (9) and forming the boundary of a lower-lying region (23; 30, 38) of the holder (9), said region being covered by the plastics sheathing (28; 36).

3. An instrument according to claim 2, **characterised in that** the recessed cavity of the undercut is completely filled with the material of the plastics sheathing (28; 36).

4. An instrument according to either one of claims 2 and 3, **characterised in that** the undercut is formed by a boundary surface (25; 32, 35, 40, 43, 45) of the step extending obliquely to the step height.

5. An instrument according to any one of the preceding claims, **characterised in that** the plastics sheathing (28; 36) steplessly adjoins unsheathed adjacent regions of the holder (9).

6. An instrument according to any one of the preceding claims, **characterised in that** the material of the plastics sheathing (28; 36) is polyether ether ketone (PEEK).

7. An instrument according to any one of claims 1 to 5, **characterised in that** the material of the plastics sheathing (28; 36) is liquid crystal polymer (LCP).

## Revendications

1. Instrument chirurgical à fût tubulaire comportant un fût (1) à l'extrémité distale duquel un outil (20) est agencé sur un support (9) qui est lui-même fixé sur le fût (1), et un enrobage de matière plastique (28 ; 36) recouvrant partiellement le support (9), **caractérisé en ce que** l'enrobage de matière plastique (28 ; 36) s'engage avec son bord au-dessous de saillies (24 ; 31, 34, 39, 41) sur le support (1).

2. Instrument selon la revendication 1, **caractérisé en ce que** les saillies sont formées par des contre-dépouilles d'un gradin (24 ; 31, 34, 39, 41) dans le support (9), qui délimite une région (23 ; 30, 38) du support (9) qui est située plus bas et qui est recouverte par l'enrobage de matière plastique (28 ; 36).

3. Instrument selon la revendication 2, **caractérisé en ce que** la cavité située en arrière de la contre-dépouille est entièrement remplie par le matériau de l'enrobage de matière plastique (28 ; 36).

4. Instrument selon l'une ou l'autre des revendications 2 et 3, **caractérisé en ce que** la contre-dépouille est formée par une surface limite (25 ; 32, 35, 40, 43, 45) du gradin, qui s'étend en biais par rapport à la hauteur du gradin.

5. Instrument selon l'une des revendications précédentes, **caractérisé en ce que** l'enrobage en matière plastique (28 ; 36) se raccorde de manière continue à des régions voisines non enrobées du support (9).

6. Instrument selon l'une des revendications précédentes, **caractérisé en ce que** le matériau de l'enrobage en matière plastique (28 ; 36) est une polyétheréthercétone (PEEK).

7. Instrument selon l'une des revendications précédentes, **caractérisé en ce que** le matériau de l'enrobage en matière plastique (28 ; 36) est un polymère à cristaux liquides (lcp).
